# EUROPEAN PATENT APPLICATION

(11) **EP 2 228 655 A1**
(43) Date of publication of application: **15.09.2010**
(21) Application number: 09075122.3
(22) Date of filing: 18.03.2009
(51) Int. Cl.: G01N 33/574, C12Q 1/68

(54) **Method of predicting metastatic potential, poor prognosis or lower overall survival of cancer patients**

(30) Priority: 10.03.2009 JP 2009056356
(71) Applicant: National Yang-Ming University, Taipei 112 (TW)
(72) Inventor: Wu, Kou-Juey, Taipei 112 (TW)
(74) Representative: Carvajal y Urquijo, Isabel

(57) **Abstract**

A method of predicting metastatic potential, poor prognosis or lower overall survival of cancer patients is provided. The method utilizes at least two reliable markers, HIF-1α, TWIST or Snail, to predict the probability of the metastatic potential, prognosis situation or overall survival of cancer patients. Moreover, the method provided by the present invention can reach relatively higher predictability of metastatic potential, prognosis situation or overall survival as compared with the current markers.

## Description

### Field of the Invention

The present invention relates to a method of predicting metastatic potential, prognosis or overall survival of cancer patients, and more particularly, to a method of predicting metastatic potential, prognosis or overall survival of cancer patients using a set of diagnostic markers.

### Background of the Invention

The current markers used to predict the metastatic potential, prognosis or overall survival of cancer patients usually did not have high predictive value or were considered controversial due to conflicting reports (Yang, M.-H., Chiang, W.-C., Chou, T.-Y., Chang, S.-Y., Chen, P.-M., Teng, S.-C. and Wu, K.-J. Increased NBS1 expression is a marker of aggressive head and neck cancer and overexpression of NBS1 contributes to transformation. Clin. Cancer Res. 2006. 12(2): 507-515). That is to say, there are still no reliable markers or a set of diagnostic markers to significantly predict the metastatic potential, prognosis or overall survival of cancer patients (Massano, J, Regateiro, F.S., Januário, G. and Ferreira, A. Oral squamous cell carcinoma: Review of prognostic and predictive factors. Oral Surg. Oral Med. Oral Pathol. Oral Radiol. Endod. 2006. 102(1): 67-76; Thomas, G. R., Nadiminti, H. and Regalado, J. Molecular predictors of clinical outcome in patients with head and neck squamous cell carcinoma. Int. J. Exp. Pathol. 2005. 86(6): 347-363. (Review).

Expression of hypoxic markers (HIF-1α, HIF-2α, and CA IX) has been repeatedly reported to correlate with the development of therapeutic resistance and worse prognosis in different types of human cancers, including breast cancer, lung caner, liver cancer, stomach cancer, kidney cancer, prostate cancer and so on (Boddy, J. L., Fox, S. B., Han, C., Campo, L., Turley, H., Kanga, S., Malone, P. R. and Harris, A. L. The androgen receptor is significantly sssociated with vascular endothelial growth factor and hypoxia sensing via hypoxia-inducible factors HIF-1α, HIF-2α, and the prolyl hydroxylases in human prostate cancer. Clin. Cancer Res. 2005. 11(21): 7658-7663; Driessen, A., Landuyt, W., Pastorekova, S., Moons, J., Goethals, L., Haustermans, K., Nafteux, P., Penninckx, F., Geboes, K., Lerut, T. and Ectors, N. Expression of carbonic anhydrase IX (CA IX), a hypoxia-related protein, rather than vascular-endothelial growth factor (VEGF), a pro-angiogenic factor, correlates with an extremely poor prognosis in esophageal and gastric adenocarcinomas. Ann. Surg. 2006. 243(3): 334-340; Giatromanolaki, A., Sivridis, E., Fiska, A., Koukourakis, M. I. Hypoxia-inducible factor-2α (HIF-2α) induces angiogenesis in breast carcinomas. Appl. Immunohistochem. Mol. Morphol. 2006. 14(1): 78-82; Giatromanolaki, A., Sivridis, E., Kouskoukis, C., Gatter, K. C., Harris, A. L. Koukourakis, M. I. Hypoxia-inducible factors 1α and 2α are related to vascular endothelial growth factor expression and a poorer prognosis in nodular malignant melanomas of the skin. Melanoma Res. 2003. 13(5): 493-501; Huang, G.-W., Yang, L.-Y. and Lu, W.-Q. Expression of hypoxia-inducible factor 1α and vascular endothelial growth factor in hepatocellular carcinoma: Impact on neovascularization and survival. World J. Gastroenterol. 2005. 11(11): 1705-1708; Kim, S. J., Rabbani, Z. N., Dewhirst, M. W., Vujaskovic, Z., Vollmer, R. T. Schreiber, E.-G., Oosterwijk, E. and Kelley, M. J. Expression of HIF-1α, CA IX, VEGF, and MMP-9 in surgically resected non-small cell lung cancer. Lung Cancer, 2005. 49(3): 325-335; Klatte, T., Seligson, D. B., Riggs, S. B., Leppert, J. T., Berkman, M. K., Kleid, M. D., Yu, H., Kabbinavar, F. F., Pantuck, A. J. and Belldegrun, A. S. Hypoxia-inducible factor 1α in clear cell renal cell carcinoma. Clin. Cancer Res. 2007. 13(24): 7388-7393, Koukourakis, M. I, Bentzen S. M., Giatromanolaki, A., Wilson, G. D., Daley, F. M., Saunders, M. I., Dische, S., Sivridis, E. and Harris, A. L. Endogenous markers of two separate hypoxia response pathways (hypoxia inducible factor 2 alpha and carbonic anhydrase 9) are associated with radiotherapy failure in head and neck cancer patients recruited in the CHART randomized trial. J. Clin. Oncol. 2006. 24(5): 727-735, Saarnio, J., Parkkila, S., Parkkila, A.-K., Pastoreková, S., Haukipuro, K., Pastorek, J., Juvonen, T. and Karttunen, T. J. Transmembrane carbonic anhydrase, MN/CA IX, is a potential biomarker for biliary tumours. J. Hepatol. 2001. 35(5): 643-649; Sumiyoshi, Y., Kakeji, Y., Egashira, A., Mizokami, K., Orita, H., and Maehara1, Y. Overexpression of hypoxia-inducible factor 1α and p53 is a marker for an unfavorable prognosis in gastric cancer. Clin. Cancer Res. 2006. 12(17): 5112-5117; Trastour, C., Benizri, E., Ettore, F., Ramaioli, A., Chamorey, E., Pouysségur J. and Berra, E. HIF-1α and CA IX staining in invasive breast carcinomas: Prognosis and treatment outcome. Int. J. Cancer, 2007, 120(7): 1451-1458; Yoshimura, H., Dhar, D. K., Kohno, H., Kubota, H., Fujii, T., Ueda, S., Kinugasa, S., Tachibana, M. and Nagasue, N. Prognostic impact of hypoxia-inducible factors 1α and 2α in colorectal cancer patients: Correlation with tumor angiogenesis and cyclooxygenase-2 expression. Clin. Cancer Res. 2004. 10(24): 8554-8560) Specifically, tumors with abundant HIF-1 stabilization through intratumoral hypoxia are more likely to develop metastasis and correlate with poor survival (Gupta, G. P. and Massagué, J. Cancer metastasis: building a framework._Cell, 2006. 127(4): 679-695. (Review); Harris, A. L. Hypoxia - a key regulatory factor in tumour growth. Nat. Rev. Cancer, 2002. 2(1): 38-47; Semenza, G. L. HIF-1 and tumor progression: Pathophysiology and therapeutics. Trends Mol. Med. 2002. 8(4): S62-S67), especially in HNSCC (Janssen, H. L., Haustermans, K. M., Balm, A. J. and Begg, A. C. Hypoxia in head and neck cancer: how much, how important? Head Neck, 2005. 27(7): 622-638. (Review) and breast cancer (Pugh, C. W., Gleadle J and Maxwell, P. H. Hypoxia and oxidative stress in breast cancer. Hypoxia signalling pathways._Breast Cancer Res. 2001. 3(5): 313-317. (Review).

Increased expression levels of EMT regulators (TWIST, Snail, Slug, SIP1, E47, Zeb1) have also been reported to be associated with an aggressive phenotype and worse prognosis in different cancers, including breast cancer, liver cancer, stomach cancer and prostate cancer (Alves, C. C., Rosivatz, E., Schott, C., Hollweck, R., Becker, I., Sarbia, M., Carneiro, F. and Becker, K.-F. Slug is overexpressed in gastric carcinomas and may act synergistically with SIP1 and Snail in the down-regulation of E-cadherin. J. Pathol. 2007. 211(5): 507-515; Blanco, M. J., Moreno-Bueno, G., Sarrio, D., Locascio, A., Cano, A., Palacios, J. and Nieto, M. A. Correlation of Snail expression with histological grade and lymph node status in breast carcinomas. Oncogene, 2002. 21(20): 3241-3246; Elloul, S., Elstrand, M. B., Nesland, J. M., Tropé, C. G., Kvalheim, G., Goldberg, I., Reich, R. and Davidson, B. Snail, Slug, and Smad-interacting protein 1 as novel parameters of disease aggressiveness in metastatic ovarian and breast carcinoma. Cancer, 2005. 103(8): 1631-1643; Foran, E., McWilliam, P., Kelleher, D., Croke, D. T., Long, A. The leukocyte protein L-plastin induces proliferation, invasion and loss of E-cadherin expression in colon cancer cells. Int. J. Cancer, 2006. 118(8): 2098-2104; Hoek, K., Rimm, D. L., Williams, K. R., Zhao, H., Ariyan, S., Lin, A., Kluger, H. M., Berger, A. J., Cheng, E., Trombetta, E. S., Wu, T., Niinobe, M., Yoshikawa, K., Hannigan, G. E. and Halaban, R. Expression profiling reveals novel pathways in the transformation of melanocytes to melanomas. Cancer Res. 2004. 64(15): 5270-5282; Lee, T. K., Poon, R. T.P., Yuen, A. P., Ling, M: T., Kwok, W. K., Wang, X. H., Wong, Y. C., Guan, X. Y., Man K., Chau, K. L. and Fan, S. T. Twist overexpression correlates with hepatocellular carcinoma metastasis through induction of epithelial-mesenchymal transition. Clin. Cancer Res. 2006. 12(18): 5369-5376; Maeda, G., Chiba, T., Okazaki, M., Satoh, T., Taya, Y., Aoba, T., Kato, K., Kawashiri, S. and Imai, K. Expression of SIP1 in oral squamous cell carcinomas: implications for E-cadherin expression and tumor progression. Int. J. Oncol. 2005. 27(6): 1535-1541; Ray, H. K., Smyrk, T. C., Koetsier, J., Victor, T. A. and Wali, R. K. The transcriptional repressor SNAIL is overexpressed in human colon cancer. Dig. Dis. Sci. 2005. 50(1): 42-46; Rosivatz, E., Becker, I., Specht, K., Fricke, E., Luber, B., Busch, R., Höfler, H. and Becker, K.-F. Differential expression of the epithelial-mesenchymal transition regulators Snail, SIP1, and Twist in gastric cancer. Am. J. Pathol. 2002. 161 (5): 1881-1891; Shih, J.-Y., Tsai, M.-F., Chang, Z.-H., Chang, Y.-L., Yuan, A., Yu, C.-J., Lin, S.-B., Liou, G.-Y., Lee, M.-L., Chen, J. J.W., Hong, T.-M., Yang, S.-C., Su, J.-L., Lee, Y.-C. and Yang, P.-C. Transcription repressor slug promotes carcinoma invasion and predicts outcome of patients with lung adenocarcinoma. Clin. Cancer Res. 2005. 11 (22): 8070-8078; Sugimachi, K., Tanaka, S., Kameyama, T., Taguchi, K., Aishima, A., Shimada, M., Sugimachi, K. and Tsuneyoshi, M. Transcriptional repressor snail and progression of human hepatocellular carcinoma. Clin. Cancer Res. 2003. 9(7): 2657-2664; Watanabe, O., Imamura, H., Shimizu, T., Kinoshita, J., Okabe, T., Hirano, A., Yoshimatsu, K., Konno, S., Aiba, M. and Ogawa, K. Expression of twist and wnt in human breast cancer. Anticancer Res. 2004. 24(6): 3851-3856; Yang, J., Mani, S. A., Donaher, J. L., Ramaswamy, S., Itzykson, R. A., Come, C., Savagner, P., Gitelman, I., Richardson A. and Weinberg, R. A. Twist, a master regulator of morphogenesis, plays an essential role in tumor metastasis. Cell, 2004. 117(7): 927-939, Yuen, H.-F., Chua, C.-W., Chan, Y.-P., Wong, Y.-C., Wang, X. and Chan, K.-W. Significance of TWIST and E-cadherin expression in the metastatic progression of prostatic cancer. Histopathology, 2007. 50(5), 648-658, Zhang, Y.-Q., Guo, X.-Y., Han, S., Chen, Yu., Ge, F.-L., Bai, F.-H., Sun, S.-R., Wei, X.-F., Ding, J. and Fan, D.-M. Expression and significance of TWIST basic helix-loop-helix protein over-expression in gastric cancer. Pathology, 2007. 39(5): 470-475). Specifically, TWIST or Snail overexpression also correlates with a worse prognosis of cancer patients (Lee, T. K., Poon, R. T.P., Yuen, A. P., Ling, M. T., Kwok, W. K., Wang, X. H., Wong, Y. C., Guan, X. Y., Man K., Chau, K. L. and Fan, S. T. Twist overexpression correlates with hepatocellular carcinoma metastasis through induction of epithelial-mesenchymal transition. Clin. Cancer Res. 2006. 12(18): 5369-5376; Moody, S. E., Perez, D., Pan, T.-C., Sarkisian, C. J., Portocarrero, C. P., Sterner, C. J., Notorfrancesco, K. L., Cardiff, R. D., and Chodosh, L. A. The transcriptional repressor Snail promotes mammary tumor recurrence. Cancer Cell, 2005. 8(3): 197-209; Yang, J., Mani, S. A., Donaher, J. L., Ramaswamy, S., Itzykson, R. A., Come, C., Savagner, P., Gitelman, I., Richardson A. and Weinberg, R. A. Twist, a master regulator of morphogenesis, plays an essential role in tumor metastasis. Cell, 2004. 117(7): 927-939, Yang, M.-H., Chang, S.-Y., Chiou, S.-H., Liu, C.-J., Chi, C.-W., Chen, P.-M., Teng, S.-C. Wu, K.-J. Overexpression of NBS1 induces epithelial-mesenchymal transition and co-expression of NBS1 and Snail predicts metastasis of head and neck cancer. Oncogene, 2007. 26(10): 1459-1467).

Despite the mentioned markers were shown as possible indicators for metastatic potential or worse prognosis of certain cancers, there are still no reports using a combination of markers such as hypoxic and EMT markers to analyze clinical samples and have a high probability of predicting prognosis and overall survival in cancer patients.

### Summary of the Invention

In consideration of the mentioned problem, it is an object of the present invention to provide a method of predicting metastatic potential of cancer patients.

It is another object of the present invention to provide at least two diagnostic markers selected from a group consisting of HIF-1α, TWIST and Snail, wherein the coexpression thereof can predict the metastatic potential of cancer patients with higher probability. The patients who co-expressed at least two diagnostic markers selected from a group consisting of HIF-1α, TWIST and Snail were predicted to have high probability of metastasis.

It is yet another object of the present invention to provide at least two diagnostic markers selected from a group consisting of HIF-1α, TWIST and Snail, wherein the coexpression thereof can be served as an indicator for poor prognosis and lower overall survival of cancer patients.

Preferably, the head and neck squamous cell carcinoma (HNSCC) patients who co-express the three markers of HIF-1α, TWIST and Snail have more than 90% probability of metastasis.

Preferably, the HNSCC patients who co-expressed the three markers of HIF-1α, TWIST and Snail were considered having the worst prognosis and requiring the extensive therapeutic intervention.

Preferably, the non-small cell lung cancer (NSCLC) patients who co-express the two markers out of HIF-1α, TWIST and Snail have more than 50% probability of predicting poor overall survival.

According to the one object of the present invention, the present invention provides a method of predicting metastatic potential of a cancer patient. The method comprises steps of obtaining a biological sample from the cancer patient; and determining if at least two diagnostic markers selected from a group consisting of HIF-1α, TWIST and Snail are co-expressed in the biological sample; wherein the coexpression of the diagnostic markers in the biological sample being indicators for the metastatic potential of the cancer patient.

Preferably, the biological sample is obtained from cancer cells.

Preferably, the cancer patient is a HNSCC patient.

Preferably, if HIF-1α, TWIST and Snail are co-expressed in biological sample of the NHSCC patient, the patient is diagnosed with higher probability of suffering the cancer metastasis.

Preferably, the cancer patient is a NSCLC patient.

The co-expression of at least two diagnostic markers selected from a group consisting of HIF-1α, TWIST and Snail can provide relatively high predictability of metastasis for HNSCC patients as compared with current effective markers.

According to another object of the present invention, the present invention provides a method of predicting prognosis or overall survival of a cancer patient. The method comprises steps of obtaining a biological sample from the cancer patient; and determining if at least two diagnostic markers selected from a group consisting of HIF-1α, TWIST and Snail are co-expressed in the biological sample; wherein the coexpression of the diagnostic markers in the biological sample being indicators for the prognosis or the overall survival of the cancer patient.

Preferably, the biological sample is obtained from cancer cells.

Preferably, the cancer patient is a HNSCC patient.

Preferably, the cancer patient is a NSCLC patient.

The co-expression of at least two diagnostic markers selected from a group consisting of HIF-1α, TWIST and Snail can be served as indicators for poor prognosis or lower overall survival for cancer patients as compared with current effective markers.

These and other objects and features of the invention will become more fully apparent when the following detailed description is read in conjunction with the accompanying figures and examples.

### Brief Description of the Drawings

Fig. 1 depicts a diagram of IHC analysis of co-expression of CA IX , HIF-1α, TWIST and Snail in corresponding normal tissue (N), primary tumor (T) and metastatic tumor (M) of a representative HNSCC case (the black arrows indicate the nuclear expression of HIF-1α, TWIST and Snail; whereas the red arrows indicate the membranous expression of CA IX;)
Fig. 2 depicts a diagram of the percentage of IHC positivity of CA IX, HIF-1α, TWIST and Snail in the N (normal tissue), T (tumor tissue) and M (metastatic tumor) samples of HNSCC cases;
Fig. 3 depicts a diagram of Kaplan-Meier analysis of metastasis-free period of HNSCC cases with HIF-1α (-) vs. HIF-1α (+), TWIST(-) vs. TWIST(+), and Snail(-) vs. Snail(+) in primary tumors of 147 HNSCC patients;
Fig. 4 depicts a diagram of probability of overall survival of HNSCC cases with HIF-1α (-) vs. HIF-1α (+), TWIST(-) vs. TWIST(+), and Snail(-) vs. Snail(+) in primary tumors of 147 HNSCC patients;
Fig. 5 depicts a diagram of Kaplan-Meier analysis of metastasis-free period of HNSCC cases with HIF-1α (-), HIF-1α (+) vs. Snail(-) vs. Twist(-), HIF-1α (+) vs. Snail(+) vs. Twist(-), HIF-1α (+) vs. Snail(-) vs. Twist (+), HIF-1α (+) vs. Snail(+) vs. Twist(+) in primary tumors of 147 HNSCC patients;
Fig. 6 depicts a diagram of probability of overall survival of HNSCC cases with with HIF-1α (-), HIF-1α (+) vs. Snail(-) vs. Twist(-), HIF-1α (+) vs. Snail(+) vs. Twist(-), HIF-1α (+) vs. Snail(-) vs. Twist (+), HIF-1α (+) vs. Snail(+) vs. Twist(+) in primary tumors of 147 HNSCC patients;
Fig. 7 depicts a diagram of a representative IHC staining of HIF-1α, TWIST and Snail markers among NSCLC patients;
Fig. 8 depicts a diagram of Kaplan-Meier analysis of survival rate of NSCLC cases with (a) HIF-1α (-) vs. HIF-1α (+), (b) TWIST(-) vs. TWIST(+), and (c) Snail(-) vs. Snail(+) in primary tumors of 87 NSCLC patients;
Fig. 9 depicts a diagram of the subgroup analysis of 87 NSCLC cases according to the expression profile of HIF-1α, TWIST, and Snail, wherein (a) shows the expression profile of HIF-1α and TWIST; (b) shows that of HIF-1α and Snail; and (c) shows that of TWIST and Snail; and

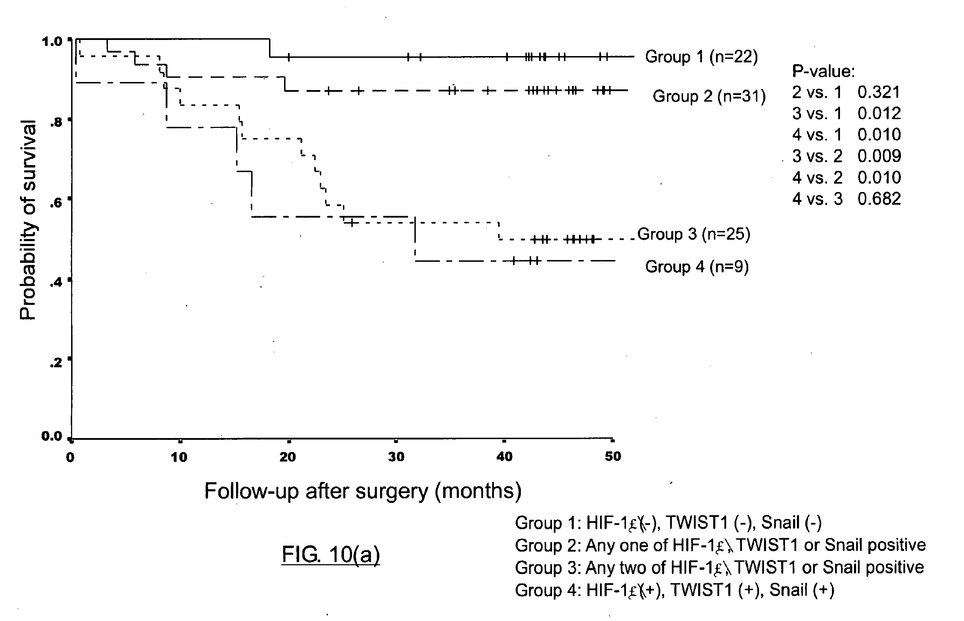

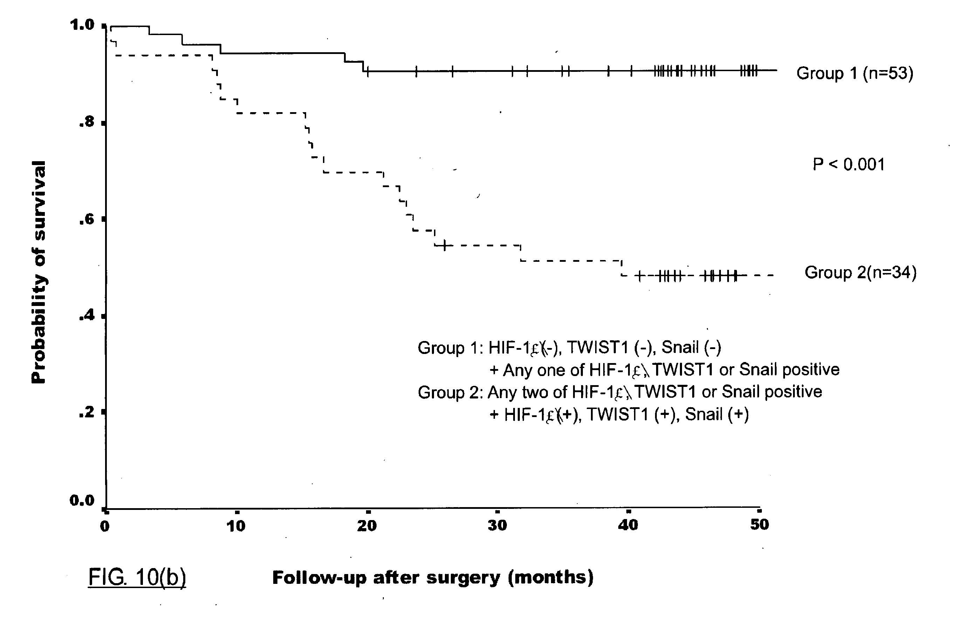

Fig. 10 depicts a diagram of Kaplan-Meier survival analysis in NSCLC patients according to the number of markers including HIF-1α, TWIST and Snail which had increased expression, wherein (a) the patients were divided into four groups: HIF-1α (-)/TWIST1(-)/Snail(-) (group1), any one of HIF-1α, TWIST1 or Snail overexpression (group 2), any two of HIF-1α, TWIST1 or Snail overexpression (group 3), and HIF-1α (+)/TWIST1(+)/Snail(+) (group 4); and (b) the patients were re-divided into two groups: None or one of HIF-1α, TWIST or Snail overexpression (group 1), and any two or all of HIF-1α, TWIST or Snail overexpression (group 2).

### Detailed Description of the Preferred Embodiment

### Embodiment 1

### A set of diagnostic markers for head and neck squamous cell carcinoma (HNSCC)

In this embodiment, tissue-microarray immunohistochemistry analysis of HIF-1α, Snail and TWIST expressions was performed (Yang, M.-H., Chang, S.-Y., Chiou, S.-H., Liu, C.-J., Chi, C.-W., Chen, P.-M., Teng, S.-C. Wu, K.-J. Overexpression of NBS1 induces epithelial-mesenchymal transition and co-expression of NBS1 and Snail predicts metastasis of head and neck cancer. Oncogene, 2007. 26(10): 1459-1467; Yang, M.-H., Wu, M.-Z., Chiou, S.-H., Chen, P.-M., Chang, S.-Y., Liu, C.-J., Teng, S.-C. and Wu, K.-J. Direct regulation of TWIST by HIF-1α promotes metastasis. Nat. Cell Biol. 2008. 10(3): 295-305).

### 1.1 Study population, sample collection and tissue microarray construction

One hundred and forty-seven HNSCC patients who underwent treatment at Taipei Mackay Memorial Hospital and Taipei Veterans General Hospital between January 2001 and December 2004 were retrospectively analyzed. This study has been approved by the Institutional Review Board of Taipei Veterans General Hospital. The clinical characteristics of 147 HNSCC patients are illustrated in Table 1.

**Table 1. Characteristics and univariate survival analysis of 147 HNSCC cases**

| Variables | | Case No. | Median OS (months) | *P* | Median RFS (months) | *P* |
|---|---|---|---|---|---|---|
| Age | | | | 0.067 | | 0.211 |
| | <50 | 60 | ---* | | ---* | |
| | ≥ 50 | 87 | 26.0 | | 28.0 | |
| Gender | | | | 0.354 | | 0.228 |
| | male | 138 | ---* | | ---* | |
| | female | 9 | ---* | | ---* | |
| T stage | | | | 0.004 | | <0.001 |
| | 1∼2 | 68 | ---* | | ---* | |
| | 3∼4 | 79 | 26 | | 18 | |
| N stage | | | | <0.001 | | <0.001 |
| | 0 | 100 | ---* | | ---* | |
| | 1-3 | 47 | 18 | | 18 | |
| HIF-1α overexpression | | | | <0.001 | | <0.001 |
| | Yes | 75 | 18 | | 14 | |
| | No | 72 | ---* | | ---* | |
| TWIST overexpression | | | | <0.001 | | <0.001 |
| | Yes | 52 | 14 | | 8 | |
| | No | 95 | ---* | | ---* | |
| Snail overexpression | | | | <0.001 | | <0.001 |
| | Yes | 55 | 14 | | 8 | |
| | No | 92 | ---* | | ---* | |

| | | | | | | |
|---|---|---|---|---|---|---|
| Abbreviations: OS, overall survival; RFS, relapse-free survival; ^{*} Median survival was not reached. | | | | | | |

Primary tumor samples and the corresponding non-cancerous matched tissue were obtained during surgery; whereas 56 metastatic tumor samples (34 of visceral metastasis and 22 of cervical nodes) were obtained when metastasis occurred. A high-density tissue microarray (TMA) was constructed as described (Yang, M.-H., Chang, S.-Y., Chiou, S.-H., Liu, C.-J., Chi, C.-W., Chen, P.-M., Teng, S.-C. Wu, K.-J. Overexpression of NBS1 induces epithelial-mesenchymal transition and co-expression of NBS1 and Snail predicts metastasis of head and neck cancer. Oncogene, 2007. 26(10): 1459-1467).

### 1.2 Immunohistochemstry (IHC)

The sample processing and IHC procedure were performed as described (Yang, M.-H., Chang, S.-Y., Chiou, S.-H., Liu, C.-J., Chi, C.-W., Chen, P.-M., Teng, S.-C. Wu, K.-J. Overexpression of NBS1 induces epithelial-mesenchymal transition and coexpression of NBS1 and Snail predicts metastasis of head and neck cancer. Oncogene, 2007. 26(10): 1459-1467). The interpretation of HIF-1α, CA IX, Snail and TWIST IHC results was performed independently by two pathologists according to the criteria described previously (Janssen, H. L., Haustermans, K. M., Balm, A. J. and Begg, A. C. Hypoxia in head and neck cancer: how much, how important? Head Neck, 2005. 27(7): 622-638. (Review); Koukourakis, M. I, Bentzen S. M., Giatromanolaki, A., Wilson, G. D., Daley, F. M., Saunders, M. I., Dische, S., Sivridis, E. and Harris, A. L. Endogenous markers of two separate hypoxia response pathways (hypoxia inducible factor 2 alpha and carbonic anhydrase 9) are associated with radiotherapy failure in head and neck cancer patients recruited in the CHART randomized trial. J. Clin. Oncol. 2006. 24(5): 727-735, Maestro, R., Dei Tos, A. P., Hamamori, Y., Krasnokutsky, S., Sartorelli, V., Kedes, L., Doglioni, C., Beach, D. H. and Hannon, G. J. Twist is a potential oncogene that inhibits apoptosis. Genes Dev. 1999. 13(17): 2207-2217; Tickoo, S. K., Alden, D., Olgac, S., Fine, S. W., Russo, P., Kondagunta, G. V., Motzer, R. J. and Reuter, V. E. Immunohistochemical expression of hypoxia inducible factor-1α and its downstream molecules in sarcomatoid renal cell carcinoma. J. Urol. 2007. 177(4): 1258-1263; Trastour, C., Benizri, E., Ettore, F., Ramaioli, A., Chamorey, E., Pouysségur J. and Berra, E. HIF-1α and CA IX staining in invasive breast carcinomas: Prognosis and treatment outcome. Int. J. Cancer, 2007, 120(7): 1451-1458; Yang, M.-H., Chang, S.-Y., Chiou, S.-H., Liu, C.-J., Chi, C.-W., Chen, P.-M., Teng, S.-C. Wu, K.-J. Overexpression of NBS1 induces epithelial-mesenchymal transition and coexpression of NBS1 and Snail predicts metastasis of head and neck cancer. Oncogene, 2007. 26(10): 1459-1467).

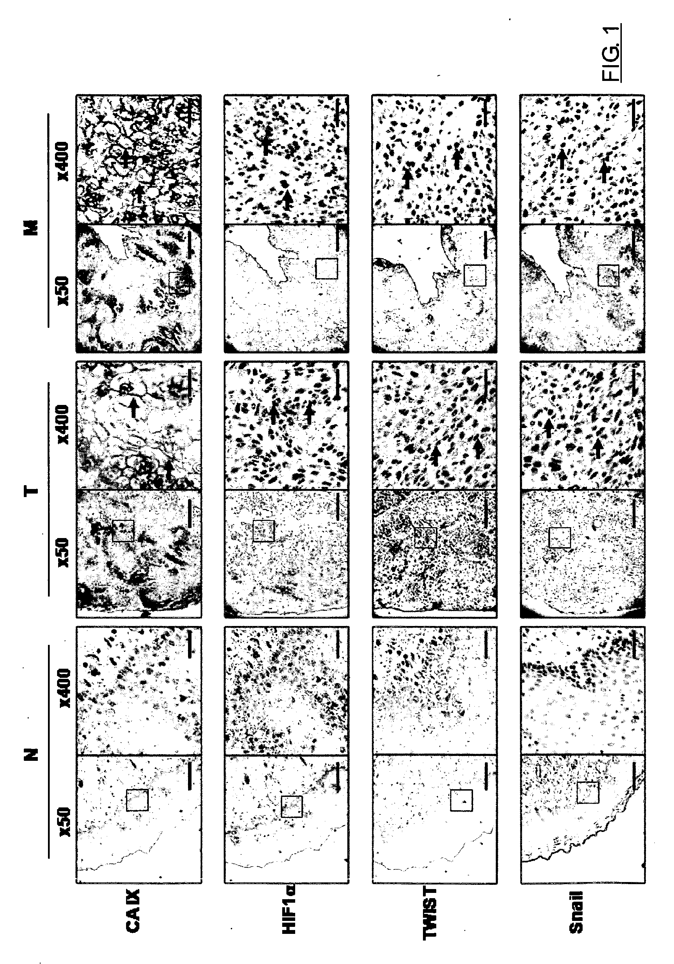

A representative case of IHC staining of all four markers was shown in Fig. 1. The functional indicator of HIF-1α, carbonic anhydrase IX (CA IX) (Hui, E. P., Chan, A. T. C., Pezzella, F., Turley, H., To, K.-F., Poon, T. C. W., Zee, B., Mo, F., Teo, P. M. L., Huang, D. P., Gatter, K. C., Johnson, P. J. and Harris, A. L. Coexpression of hypoxia-inducible factors 1α and 2α, carbonic anhydrase IX, and vascular endothelial growth factor in nasopharyngeal carcinoma and relationship to survival. Clin. Cancer Res. 2002. 8(8): 2595-2604; Koukourakis, M. I, Bentzen S. M., Giatromanolaki, A., Wilson, G. D., Daley, F. M., Saunders, M. I., Dische, S., Sivridis, E. and Harris, A. L. Endogenous markers of two separate hypoxia response pathways (hypoxia inducible factor 2 alpha and carbonic anhydrase 9) are associated with radiotherapy failure in head and neck cancer patients recruited in the CHART randomized trial. J. Clin. Oncol. 2006. 24(5): 727-735; Trastour, C., Benizri, E., Ettore, F., Ramaioli, A., Chamorey, E., Pouysségur J. and Berra, E. HIF-1α and CA IX staining in invasive breast carcinomas: Prognosis and treatment outcome. Int. J. Cancer, 2007, 120(7): 1451-1458), was stained in all samples to further correlate the hypoxic zones of tumors. The result showed that tumors with ≥50% hypoxic areas (CA IX ≥50% staining) significantly correlated with HIF-1α overexpression (≥50% HIF-1α nuclear expression in tumor cells) (P=0.001, data not shown). It was confirmed that there is good correlation between HIF-1α and CA IX IHC results, which is consistent with previous reports (Hui, E. P., Chan, A. T. C., Pezzella, F., Turley, H., To, K.-F., Poon, T. C. W., Zee, B., Mo, F., Teo, P. M. L., Huang, D. P., Gatter, K. C., Johnson, P. J. and Harris, A. L. Coexpression of hypoxia-inducible factors 1α and 2α, carbonic anhydrase IX, and vascular endothelial growth factor in nasopharyngeal carcinoma and relationship to survival. Clin. Cancer Res. 2002. 8(8): 2595-2604; Koukourakis, M. I, Bentzen S. M., Giatromanolaki, A., Wilson, G. D., Daley, F. M., Saunders, M. I., Dische, S., Sivridis, E. and Harris, A. L. Endogenous markers of two separate hypoxia response pathways (hypoxia inducible factor 2 alpha and carbonic anhydrase 9) are associated with radiotherapy failure in head and neck cancer patients recruited in the CHART randomized trial. J. Clin. Oncol. 2006. 24(5): 727-735; Trastour, C., Benizri, E., Ettore, F., Ramaioli, A., Chamorey, E., Pouysségur J. and Berra, E. HIF-1α and CA IX staining in invasive breast carcinomas: Prognosis and treatment outcome. Int. J. Cancer, 2007, 120(7): 1451-1458).

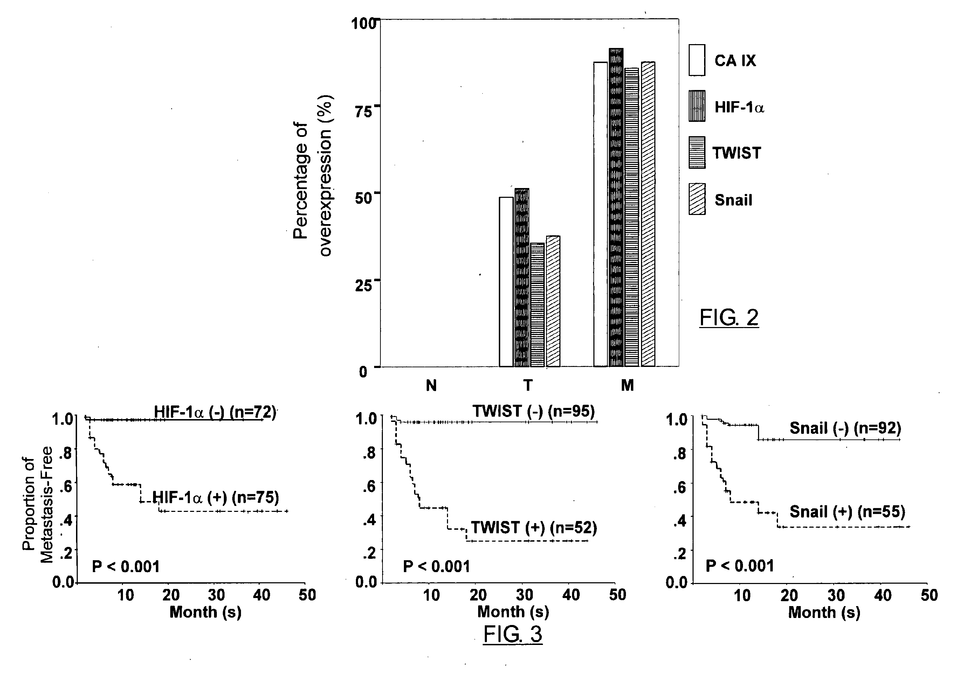

Figure 2 depicts the percentage of IHC positivity of CA IX, HIF-1α, TWIST and Snail in the N (normal tissue), T (tumor tissue), and M (metastatic tumor) samples of HNSCC cases. This result also identified the patient group with hypoxic tumors (48.3% of primary and 91.1% of metastatic HNSCCs). TWIST and Snail overexpression (≥50% nuclear expression in tumor cells) was shown in 35.4% and 37.4% of primary tumors and in 85.7% and 82.1% of metastatic ones, respectively. Considering the correlation between tumor hypoxia or HIF-1α overexpression and activation of TWIST or Snail, tumors with more than 50% hypoxic areas significantly correlated with TWIST or Snail overexpression (a representative case shown in Fig. 1; *P*<0.001, 0.001, respectively) (data not shown).

The mentioned results indicated that tumor hypoxia correlated with HIF-1α overexpression and consequentially with TWIST and Snail expression. In addition, a higher proportion of HIF-1α, TWIST, or Snail expression was observed in metastatic tumor samples than in primary ones (Fig. 2).

### 1.3 The intensity of HIF-1α expression correlates with the respective expression of TWIST and Snail

The immunoreactivity of HIF-1α, TWIST and Snail was graded from 0 to 3⁺ (0, no staining; 1⁺, 1∼25%; 2⁺, 26∼50%; 3⁺, >50% nuclear staining) according to nuclear expression, and only 3⁺ (>50% nuclear staining) was considered as a positive IHC result (Hui, E. P., Chan, A. T. C., Pezzella, F., Turley, H., To, K.-F., Poon, T. C. W., Zee, B., Mo, F., Teo, P. M. L., Huang, D. P., Gatter, K. C., Johnson, P. J. and Harris, A. L. Coexpression of hypoxia-inducible factors 1α and 2α, carbonic anhydrase IX, and vascular endothelial growth factor in nasopharyngeal carcinoma and relationship to survival. Clin. Cancer Res. 2002. 8(8): 2595-2604; Koukourakis, M. I, Bentzen S. M., Giatromanolaki, A., Wilson, G. D., Daley, F. M., Saunders, M. I., Dische, S., Sivridis, E. and Harris, A. L. Endogenous markers of two separate hypoxia response pathways (hypoxia inducible factor 2 alpha and carbonic anhydrase 9) are associated with radiotherapy failure in head and neck cancer patients recruited in the CHART randomized trial. J. Clin. Oncol. 2006. 24(5): 727-735; Maestro, R., Dei Tos, A. P., Hamamori, Y., Krasnokutsky, S., Sartorelli, V., Kedes, L., Doglioni, C., Beach, D. H. and Hannon, G. J. Twist is a potential oncogene that inhibits apoptosis. Genes Dev. 1999. 13(17): 2207-2217; Tickoo, S. K., Alden, D., Olgac, S., Fine, S. W., Russo, P., Kondagunta, G. V., Motzer, R. J. and Reuter, V. E. Immunohistochemical expression of hypoxia inducible factor-1α and its downstream molecules in sarcomatoid renal cell carcinoma. J. Urol. 2007. 177(4): 1258-1263, Trastour, C., Benizri, E., Ettore, F., Ramaioli, A., Chamorey, E., Pouysségur J. and Berra, E. HIF-1α and CA IX staining in invasive breast carcinomas: Prognosis and treatment outcome. Int. J. Cancer, 2007, 120(7): 1451-1458; Yang, M.-H., Chang, S.-Y., Chiou, S.-H., Liu, C.-J., Chi, C.-W., Chen, P.-M., Teng, S.-C. Wu, K.-J. Overexpression of NBS1 induces epithelial-mesenchymal transition and co-expression of NBS1 and Snail predicts metastasis of head and neck cancer. Oncogene, 2007. 26(10): 1459-1467).

The correlation of the IHC expression gradient of HIF-1α, TWIST and Snail in primary tumor samples of 147 HNSCC cases is illustrated in Table 2, which indicated that the expression gradient of HIF-1α also correlated significantly with that of TWIST or Snail (P<0.001, <0.001, respectively).

**Table 2. Correlation of the IHC expression gradient of HIF-1α, TWIST, and Snail in primary tumor samples of 147 HNSCC cases**

| | | HIF-1α IHC gradient | | | | *P* |
|---|---|---|---|---|---|---|
| | | 0 | 1 | 2 | 3 | |
| TWIST | 0 | 33 | 10 | 0 | 9 | <0.001 |
| IHC | 1 | 1 | 9 | 2 | 6 | |
| gradient | 2 | 0 | 3 | 12 | 10 | |
| | 3 | 0 | 0 | 2 | 50 | |
| Snail | 0 | 32 | 8 | 3 | 0 | <0.001 |
| IHC | 1 | 0 | 12 | 1 | 18 | |
| gradient | 2 | 0 | 0 | 5 | 13 | |
| | 3 | 2 | 2 | 7 | 44 | |

### 1.4 Statistical analysis

The independent Student's t-test was used to compare the continuous variables between two groups, and the χ² test was applied for comparison of dichotomous variables. The Kaplan-Meier estimate was used for metastasis-free and overall survival analysis, and the log-rank test was used to compare the difference. The Cox's proportional hazards model was applied in multivariate survival analysis to test independent prognostic factors. The control groups of all the statistical analyses were usually the first groups in the panels unless specified otherwise in the figure legends. The level of statistical significance was set at 0.05 for all tests.

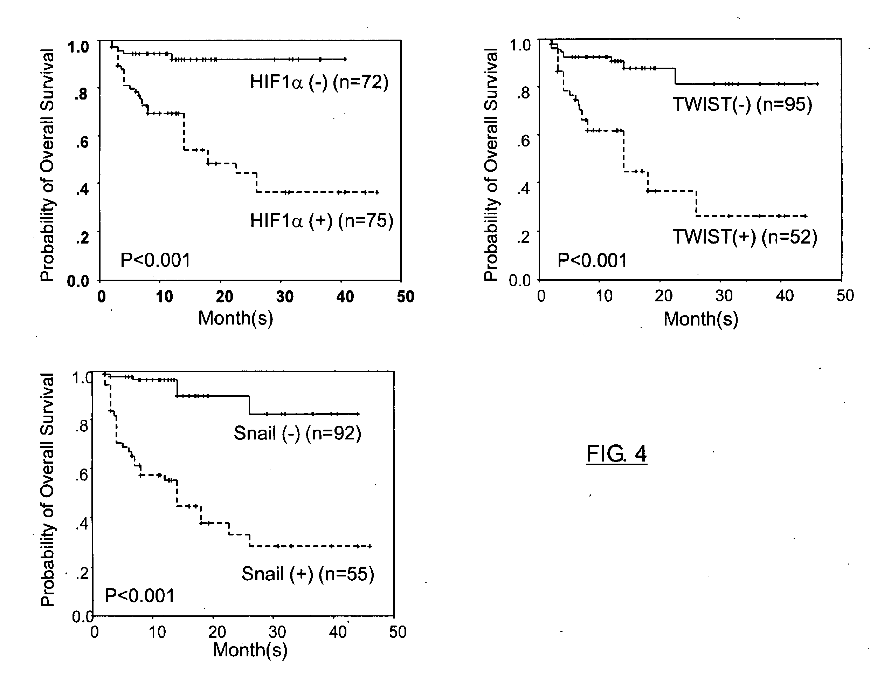

There was no statistical difference in the expression pattern of HIF-1α, TWIST, and Snail among the metastatic samples obtained from cervical nodes or visceral organ (data not shown). For metastasis and survival analysis, overexpression of HIF-1α, TWIST or Snail in primary HNSCCs was associated with a shorter metastasis-free period (Fig. 3), and the same situation was observed in the overall survival analysis (P<0.001, <0.001, <0.001, respectively) (Fig. 4 ). It is shown that the respective overexpression of HIF-1α, TWIST and Snail in HNSCC patients could result in lower probability of overall survival.

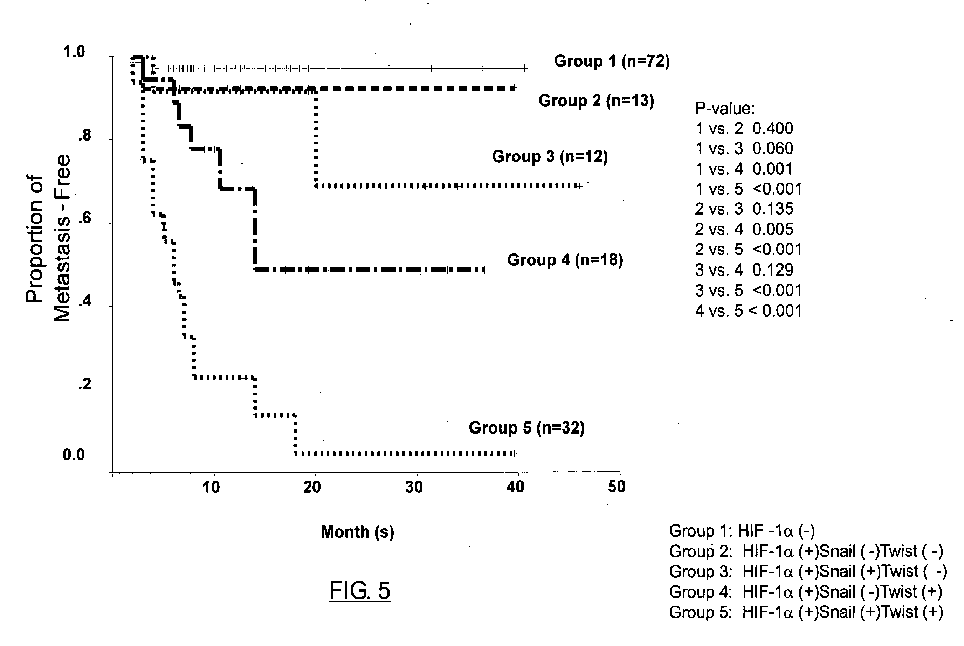

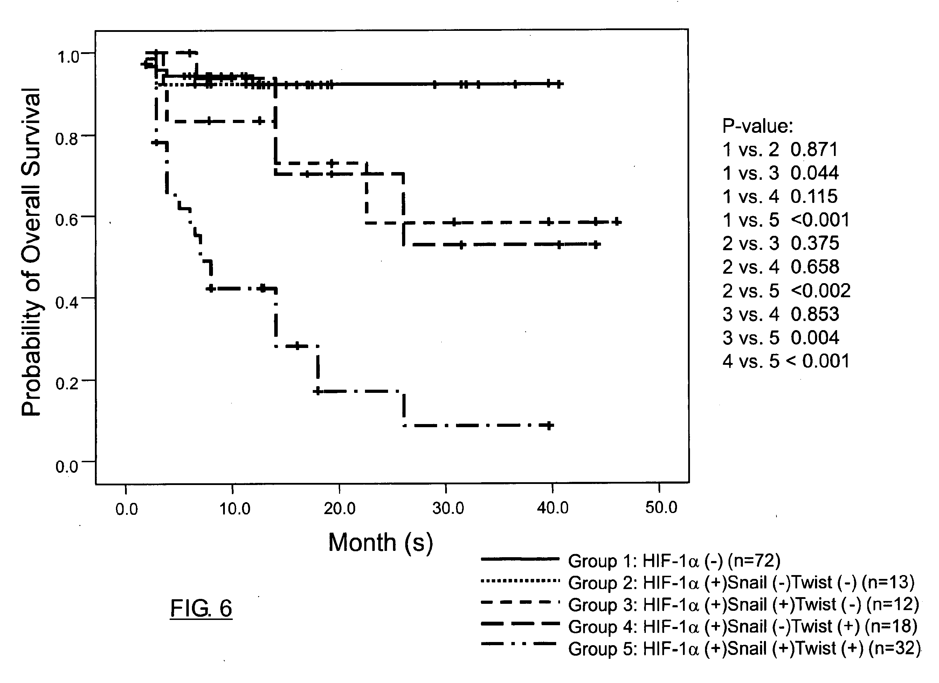

Furthermore, to investigate the prognostic significance of the expression pattern of HIF-1α, TWIST, and Snail in HNSCCs, HNSCC patients were divided into five groups based on the expression profile of HIF-1α, TWIST and Snail: (1) HIF-1α(-); (2) HIF-1α(+)Snail(-)TWIST(-); (3) HIF-1α(+)Snail(+)TWIST(-); (4) HIF-1α(+)Snail(-)TWIST(+); and (5) HIF-1α(+)Snail(+)TWIST(+). Kaplan-Meier metastasis-free curves were generated and the log-rank test was used to test for significant difference among groups. The results showed that the groups 3 and 4 have the shorter metastasis-free period and co-expression of HIF-1α, TWIST, and Snail (Group 5) exhibited the shortest metastasis-free period as compared therewith (Fig. 5). That is, more than 90% patients of Group 5 had been diagnosed with metastasis. Similar pattern was observed in the overall survival analysis, which also showed that more than 90% patients of Group 5 died (Fig. 6). The mentioned results demonstrated that activation of TWIST and/or Snail by HIF-1α to promote metastasis indeed occurred in HNSCC patients and co-expression of HIF-1α, TWIST and Snail was associated with the most aggressive outcome.

In this embodiment, it was evident that the activation of TWIST and/or Snail by HIF-1α indeed occurs in HNSCC cancers and the expression profile of HIF-1α, TWIST and Snail could be used as an evaluative indicator for the prognostic significance of cancer patients. That is, co-expression of HIF-1α, TWIST, and Snail could be used as a valuable marker to predict metastasis and prognosis or overall survival in HNSCC patients.

### Embodiment 2

### A predictive marker set for non-small Cell Lung Cancer (NSCLC)

HIF-1α overexpression was noted in 62% of primary NSCLC tumors, and was marginally associated with poor prognosis (Giatromanolaki, A., Koukourakis, M. I., Sivridis, E., Turley, H., Talks, K., Pezzella, F., Gatter, K. C. and Harris, A. L. Relation of hypoxia inducible factor 1α and 2α in operable non-small cell lung cancer to angiogenic/molecular profile of tumours and survival. Br. J. Cancer, 2001. 85(6): 881-890). HIF-1α mRNA expression was shown to be prognostic in early-stage NSCLC (Lau, S. K., Boutros, P. C., Pintilie, M., Blackhall, F. H., Zhu, C.-Q., Strumpf, D., Johnston, M. R., Darling, G., Keshavjee, S., Waddell, T. K., Liu, N., Lau, D., Penn, L. Z., Shepherd, F. A., Jurisica, I., Der, S. D. and Tsao, M.-S. Three-gene prognostic classifier for early-stage non-small-cell lung cancer. J. Clin. Oncol. 2007. 25(35): 5562-5569). In the embodiment, to investigate the relationship among HIF-1α, TWIST and Snail in lung cancer cells, tissue-microarray immunohistochemistry analysis of HIF-1α, TWIST and Snail expressions was performed.

### 2.1 Study population, sample collection and tissue microarray construction

Eighty-seven NSCLC patients who underwent surgical resection at Taipei Mackay Memorial Hospital and Taipei Veterans General Hospital between January 2003 and December 2004 were retrospectively analyzed. This study has been approved by the Institutional Review Board of Taipei Veterans General Hospital. The clinical characteristics of 87 NSCLC patients are illustrated in Table 3.

**Table 3. Characteristics and univariate survival analysis of 87 NSCLC patients**

| Variables | | Case No. | Median OS (months) | *P* | HIF-1□, TWIST , Snail expression | | |
|---|---|---|---|---|---|---|---|
| | | | | | None or one (%) (n=53) | Two or three (%) (n=34) | *P* |
| Age | | | | 0.856 | | | 0.550 |
| | <65 | 25 | ---* | | 14 (56.0) | 11 (44.0) | |
| | ≥ 65 | 62 | 53.5 | | 39 (62.9) | 23 (37.1) | |
| Gender | | | | 0.371 | | | 0.600 |
| | male | 69 | 53.5 | | 43 (62.3) | 26 (37.7) | |
| | female | 18 | ---* | | 10 (55.6) | 8 (44.4) | |
| T stage | | | | 0.004 | | | 0.752 |
| | 1∼2 | 73 | ---* | | 45 (61.6) | 28 (38.4) | |
| | 3∼4 | 14 | 23 | | 8(57.1) | 6 (42.9) | |
| N stage | | | | 0.012 | | | 0.947 |
| | 0 | 49 | ---* | | 30 (61.2) | 19 (38.3) | |
| | 1-3 | 38 | 53.5 | | 23 (60.5) | 15 (39.5) | |
| Histological | | | | 0.213 | | | 0.063 |
| type | | | | | | | |
| Adenocarcinoma | | 54 | 53.5 | | 37 (68.5) | 17 (31.5) | |
| Non-adenocarcinoma | | 33 | | | 16 (48.5) | 17 (51.5) | |
| Extent of pulmonary reseciton | | | | 0.09 | | | 0.154 |
| Lobectomy or widget resection | | 79 | | | 50 (63.3) | 29 (36.7) | |
| Pneumonectomy or bilobectomy | | 8 | 16.6 | | 3 (37.5) | 5 (62.5) | |
| HIF-1α overexpression | | | | 0.03 | | | |
| | Yes | 28 | ---* | | | | |
| | No | 59 | ---* | | | | |
| TWIST | | | | 0.021 | | | |
| overexpression | | | | | | | |
| | Yes | 32 | ---* | | | | |
| | No | 55 | ---* | | | | |
| Snail | | | | 0.004 | | | |
| overexpression | | | | | | | |
| | Yes | 48 | 53.3 | | | | |
| | No | 39 | ---* | | | | |

| | | | | | | | |
|---|---|---|---|---|---|---|---|
| ^{*} Median survival was not reached. | | | | | | | |

### 2.2 Immunohistochemstry (IHC)

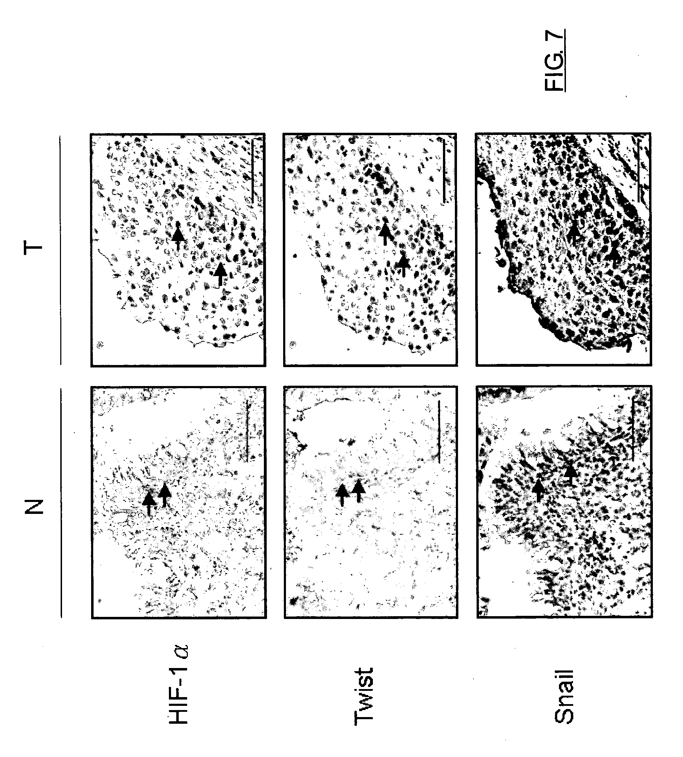

The same procedures were performed as in Embodiment 1. The immunoreactivity of HIF-1α, Twist and Snail was graded from 0 to 3⁺ (0, no staining; 1⁺, 1∼25% nuclear staining; 2⁺, 26∼50% nuclear staining; 3⁺, >50% nuclear staining) and only 3⁺ (>50% nuclear staining) was considered as a positive IHC result. A representative case of IHC staining of all markers in NSCLC was shown in Fig. 7.

According to Table 3, the overexpression profiles of HIF-1α, Twist and Snail in 87 NSCLC specimens by IHC were 28 / 87 (32.2%), 32 / 87 (36.8%) and 48 / 87 (55.2%), respectively.

### 2.3 Statistical analysis

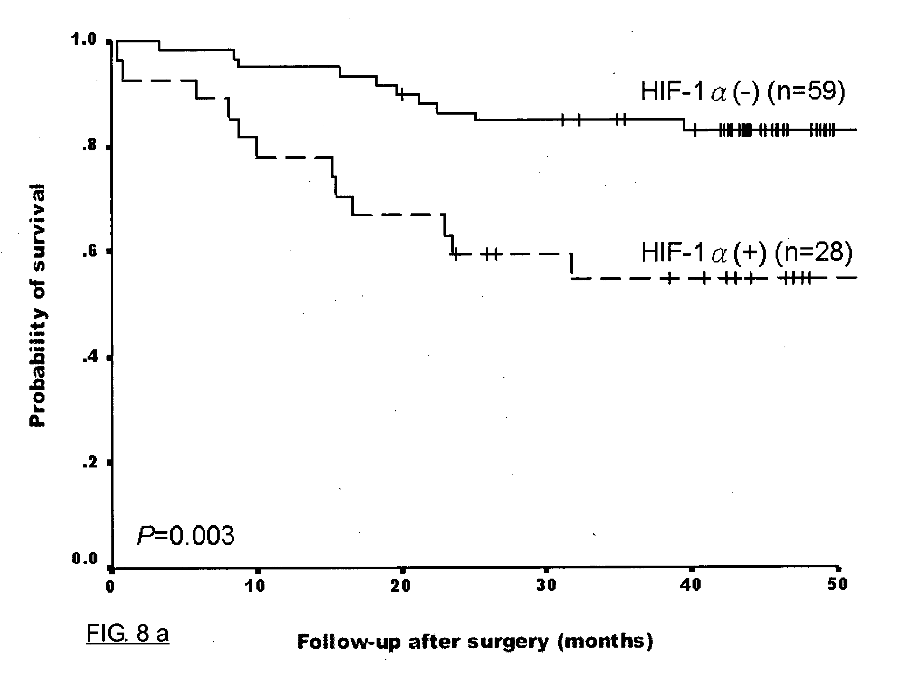

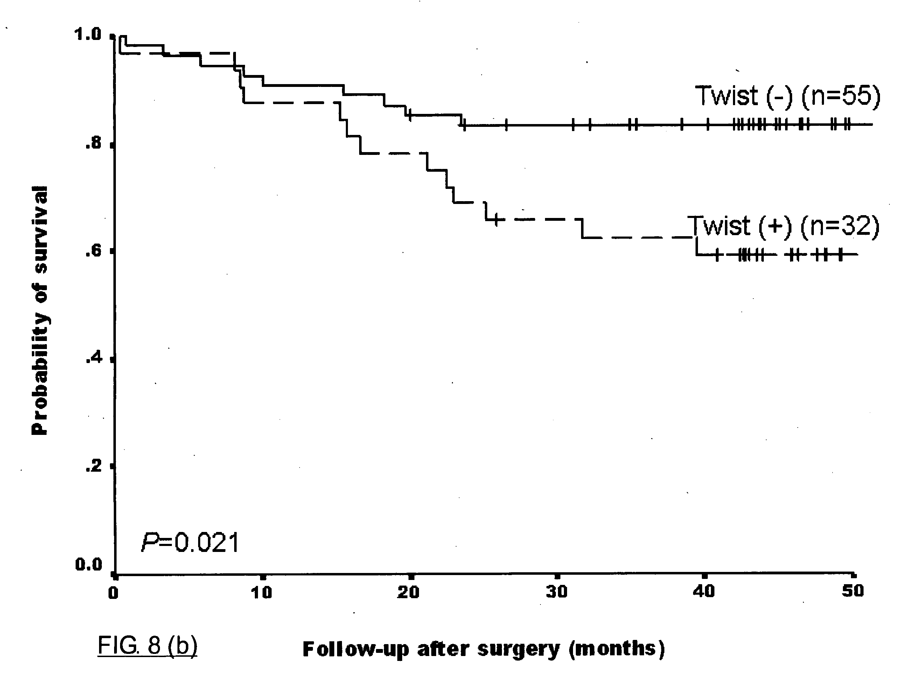

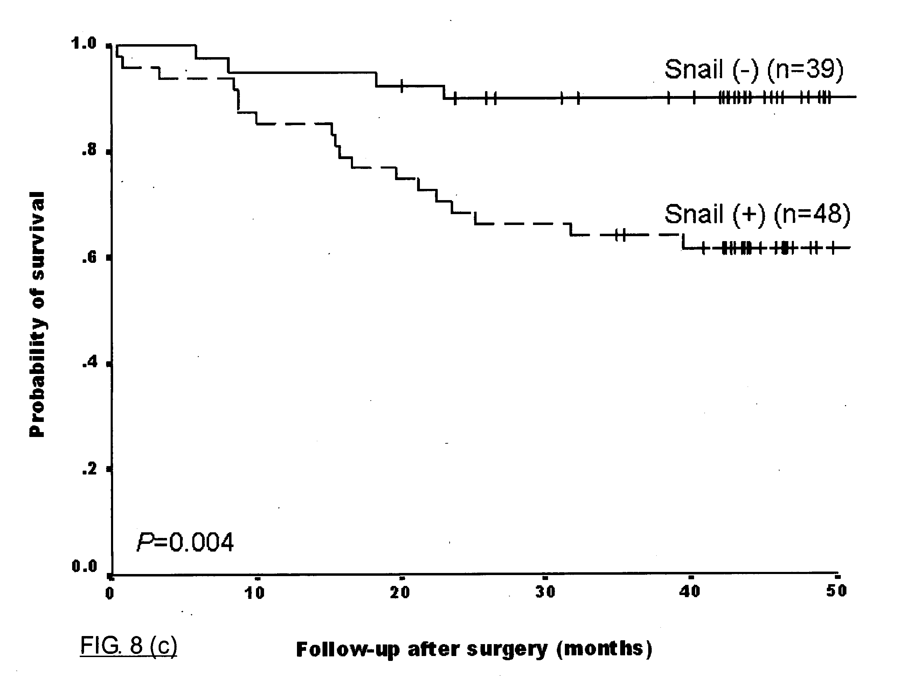

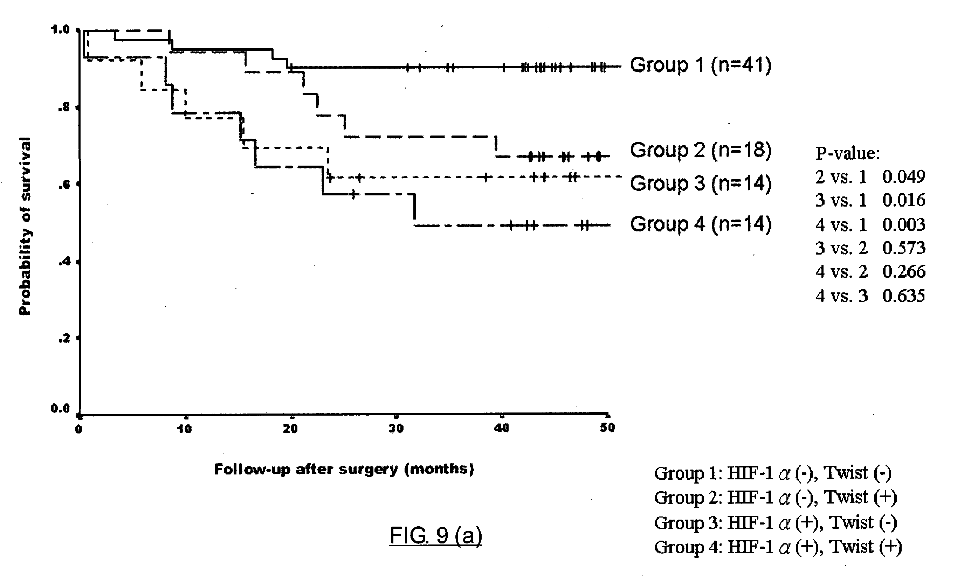

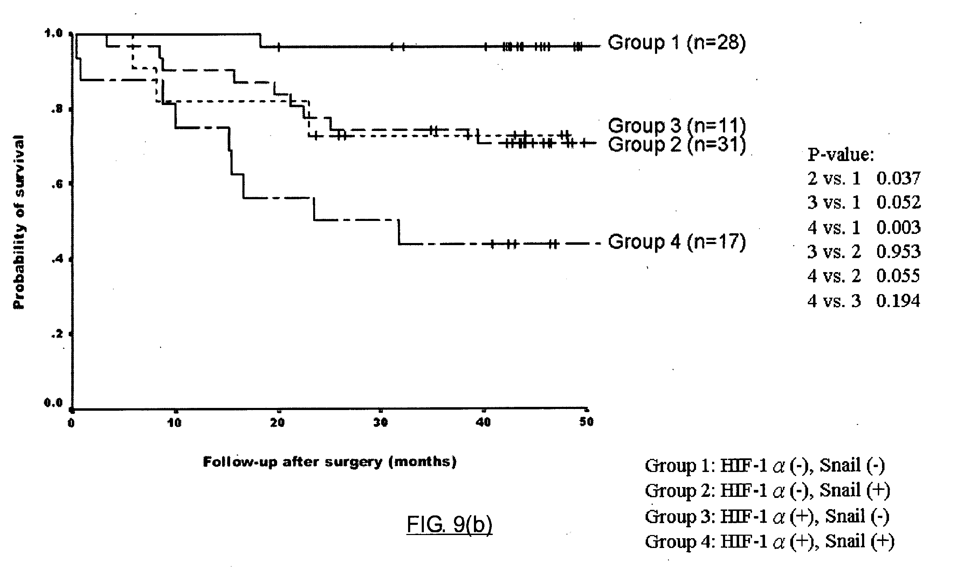

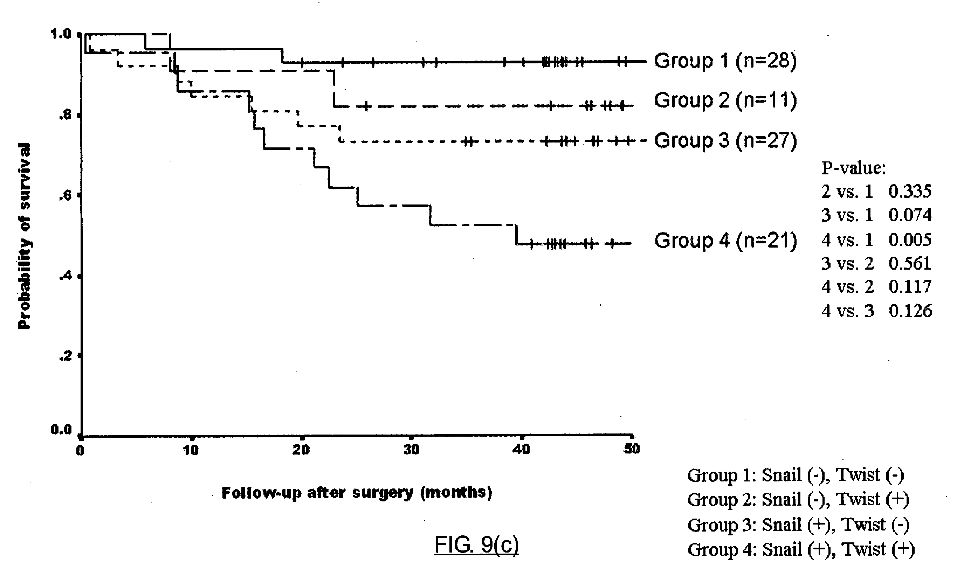

The same statistical methods to the embodiment 1 were used herein. For survival analysis shown in Figs. 8(a)-(c), the respective overexpression of HIF-1α, TWIST and Snail in NSCLCs were associated with a shorter overall survival. Furthermore, to investigate the prognostic significance of the co-expression pattern of any two of HIF-1α, TWIST, and Snail markers in NSCLCs, subgroup analysis of NSCLC cases was shown in Figs 9(a)-(c). From Fig. 10(a), any two of HIF-1α, TWIST or Snail overexpression (Group 3) and HIF-1α (+)/TWIST1(+)/Snail(+) (group 4) had a shorter overall survival when compared with the other groups. Further, from Fig. 10(b), co-expression of any two or all of HIF-1α, TWIST and Snail (Group 2) had a significantly worse overall survival. Therefore, based on the result of Fig. 10, coexpression of two or three markers out of HIF-1α, TWIST and Snail had a worse prognosis and overall survival than no-expression or expression of one marker.

In this embodiment, it was also evident that the activation of TWIST and/or Snail by HIF-1α indeed occurs in NSCLC cancers and co-expression of at least two markers out of HIF-1α, TWIST and Snail could be used as evaluative indicators for the prognostic significance thereof.

In the present invention the IHC technique was used to analyze the situation of cancer patients, but the same result can be obtained by other kinds of analytical techniques, such as RT-PCR or Real-time PCR, and the scope of the present invention, therefore, should not be limited thereto.

Based on the above, the present invention provides at least two diagnostic markers selected from a group consisting of HIF-1α, TWIST and Snail as a set of reliable markets to predict the metastatic potential, the prognosis or the overall survival of cancer patients.

It can be known from the aforementioned preferred embodiment of the present invention, as is understood by a person skilled in the art, the foregoing preferred embodiments of the present invention are illustrated of the present invention rather than limiting of the present invention. It is intended to cover various modifications and similar arrangements included within the spirit and scope of the appended claims, the scope of which should be accorded the broadest interpretation so as to encompass all such modifications and similar structure.

## Claims

1. A method of predicting metastatic potential of a cancer patient, comprising:
obtaining a biological sample from the cancer patient; and
determining if at least two diagnostic markers selected from a group consisting of HIF-1α, TWIST and Snail, are co-expressed in the biological sample; and
wherein the co-expression of the diagnostic markers in the biological sample being indicators for the metastatic potential of the cancer patient.

2. The method of Claim 1, wherein the cancer patent is a head and neck squamous cell carcinoma (HNSCC) patient.

3. The method of Claim 2, wherein the diagnostic markers includes HIF-1α, TWIST and Snail.

4. The method of Claim 1, wherein the cancer patient is a non-small cell lung cancer (NSCLC) Patient.

5. The method of Claim 1, wherein the biological sample is obtained from cancer cells.

6. A method of predicting prognosis or overall survival of a cancer patient, comprising:
obtaining a biological sample from the cancer patient; and
determining if at least two diagnostic markers selected from a group consisting of HIF-1α, TWIST and Snail, are co-expressed in the biological sample; and
wherein the co-expression of the diagnostic markers in the biological sample being indicators for the prognosis or the overall survival of the cancer patient.

7. The method of Claim 6, wherein the cancer patient is a head and neck squamous cell carcinoma (HNSCC) patient.

8. The method of Claim 7, wherein the diagnostic markers includes HIF-1α, TWIST and Snail.

9. The method of Claim 6, wherein the cancer patient is a non-small cell lung cancer (NSCLC) patient.

10. The method of Claim 9, wherein the biological sample is obtained from cancer cells.
